# EUROPEAN PATENT APPLICATION

(11) **EP 1 118 668 A1**
(43) Date of publication of application: **25.07.2001**
(21) Application number: 00100351.6
(22) Date of filing: 07.01.2000
(51) Int. Cl.: C12N 15/62, C12N 9/00, C12N 5/10, C12N 1/21, C07K 14/435, C07K 14/035, C07K 14/16, A01K 67/027, A61K 38/43, A61K 47/48

(54) **Transduction of recombinases for inducible gene targeting**

(71) Applicant: ARTEMIS Pharmaceuticals GmbH, 51063 Köln (DE)
(72) Inventor: Schwenk, Frieder, 50739 Köln (DE)
(74) Representative: Helbing, Jörg, Dr.Dipl.-Chem.

(57) **Abstract**

The present invention provides the use of a fusion protein comprising a site-specific DNA recombinase domain and a protein transduction domain for preparing an agent for inducing target gene alteration in a living organism, suitable fusion proteins and a method for the production of said fusion proteins.

## Description

The present invention provides the use of a fusion protein comprising a site-specific DNA recombinase domain and a protein transduction domain for preparing an agent for inducing target gene alteration in a living organism, suitable fusion proteins and a method for the production of said fusion proteins.

### Background

For some years targeted mutagenesis in totipotent mouse embryonic stem (ES) cells has been used to inactivate genes, for which cloned sequences were available (Capecchi Trends in Genetics 5, 70 - 76 (1989)). Since ES cells can pass mutations induced in vitro to transgenic offspring in vivo, it is possible to analyze the consequences of gene disruption in the context of the entire organism. Thus, numerous mouse strains with functionally inactivated genes ("knock out mice") have been created by this technology and utilized to study the biological function of a variety of genes.

A refined method of targeted mutagenesis, referred to as conditional mutagenesis, employs a site-specific recombination system (e.g. Cre/loxP or Flp/frt - Sauer and Henderson, N. Proc. Natl. Acad. Sci. USA 85, 5166-5170 (1988); Senecoff et al., J. Mol. Biol., 201, 405 - 421 (1988)) which enables a temporally and/or spatially restricted alteration of target genes (Rajewsky et al, J. Clin. Invest., 98, 600 - 603 (1996)). The creation of conditional mouse mutants requires the generation of two mouse strains, i.e. the recombinase recognition strain and the recombinase expressing strain. The recombinase recognition strain is generated by homologous recombination in ES cells as described above except that the targeted exon(s) is (are) flanked by two recombinase recognition sequences (hereinafter "RRS"; e.g. loxP or frt). The type of recombination event mediated by the recombinase depends on the disposition of the RRS, with deletions, inversions, translocations and integrations being possible (Torres and Kühn, Oxford Univerity Press, Oxford, New York (1997)). By placing the RRS into introns, an interference with gene expression before recombination can be avoided. The recombinase expressing strain contains a recombinase transgene (e.g. Cre, Flp) whose expression is either restricted to certain cells and tissues or is inducible by external agents. Crossing of the recombinase recognition strain with the recombinase expressing strain recombines the RRS-flanked exons from the doubly transgenic offspring in a prespecified temporally and/or spatially restricted manner. Thus, the method allows the temporal analysis of gene function in particular cells and tissues of otherwise widely expressed genes. Moreover, it enables the analysis of gene function in the adult organism by circumventing embryonic lethality which is frequently the consequence of gene mutation. For pharmaceutical research, aiming to validate the utility of genes and their products as targets for drug development, inducible mutations provide an excellent genetic tool. However, the current systems for inducible recombinase expression in transgenic animals suffer from a certain degree of leakyness in the absence of the inducer (Kühn et al., Science 269(5229):1427-9 (1995); Schwenk et al., Nucleic Acids Res; 26(6):1427-32 (1998)). Furthermore, the generation of conditional mutants is a time consuming and labor intensive procedure, since the recombinase recognition strain and the recombinase expressing strain have to be breed at least over two generations in order to obtain animals carrying both, the recombinase transgene and two copies of the RRS-flanked target gene sequence.

Protein domains that have the ability to cross cell membranes were identified in the Antennapedia protein from Drosophila (Vives et al., J Biol Chem, 272(25):16010-7 (1997)), VP22 from HSV (Elliott and O'Hare, Cell, 88(2):223-33 (1997)) and TAT from HIV (Green and Loewenstein, Cell, 55(6):1179-88 (1988); Frankel and Pabo, Cell, 55(6):1189-93 (1988)). Fusion of such domains to heterologous proteins conferred the ability to transduce into cultured cells (Fawell et al., Proc Natl Acad Sci U S A, 91(2):664-8 (1994); Elliott and O'Hare (1997), Phelan et al., Nature Biotech. 16; 440-443 (1998) and Dilber et al., Gene Ther., 6(1):12-21 (1999)). Dalby and Bennett showed that a fusion protein consisting of VP22 and functional Flp recombinase translocated between cells in culture (from COS-1 cells transfected with VP22-Flp to CHO cells carrying Flp recognition sites (FRT sites); see Dalby and Bennett, Invitrogen, Expressions 6.2, page 13 (1999)).

On the other hand, a recent report demonstrated that the ß-galactosidase protein fused to the 11 amino acids transduction domain from the HIV TAT protein can infiltrate all tissues of living mice reaching every single cell (Schwarze et al., Science, 285(5433):1569-72 (1999)).

It was found that site-specific DNA recombinases can be translocated into cells of a living organism when fused to a protein transduction domain. Thus, whenever a gene mutation is desired, recombination is induced upon the injection of the appropriate site-specific recombinase fused to a transduction domain into such a living organism (provided, however, that said organism carries at least one appropriate RRS integrated in the genome).

The present invention thus provides
(1) the use of a fusion protein comprising
   (a) a site-specific DNA recombinase domain and
   (b) a protein transduction domain
      for preparing an agent for inducing target gene alterations in a living organism, wherein said living organism carries at least one or more recognition sites for said site-specific DNA recombinase integrated in its genome;
(2) a method for inducing gene alterations in a living organism which comprises administering to said living organism a fusion protein comprising a site-specific DNA recombinase domain and a protein transduction domain as defined in (1) above, wherein said living organism carries at least one or more recognition sites for said site-specific DNA recombinase integrated in its genome;
(3) a fusion protein comprising
   (a) a site-specific DNA recombinase domain and
   (b) a protein transduction domain
      provided that when the site-specific DNA recombinase domain is wild type Flp then the protein transduction domain is not the VP22 protein of HSV (i.e., the fusion protein is not identical to the fusion protein of Dalby and Bennett (1999));
(4) a DNA sequence coding for the fusion protein of (3) above;
(5) a vector comprising the DNA sequence as defined in (4) above;
(6) a host cell transformed with the vector of (5) above and/or comprising the DNA of (4) above;
(7) a method for producing the fusion protein of (1) above which comprises culturing the transformed host cell of (6) above and isolating the fusion protein; and
(8) an injectable composition comprising the fusion protein as defined in (1) or (3) above.

The invention is further illustrated by the appended Figure and is explained in detail below.

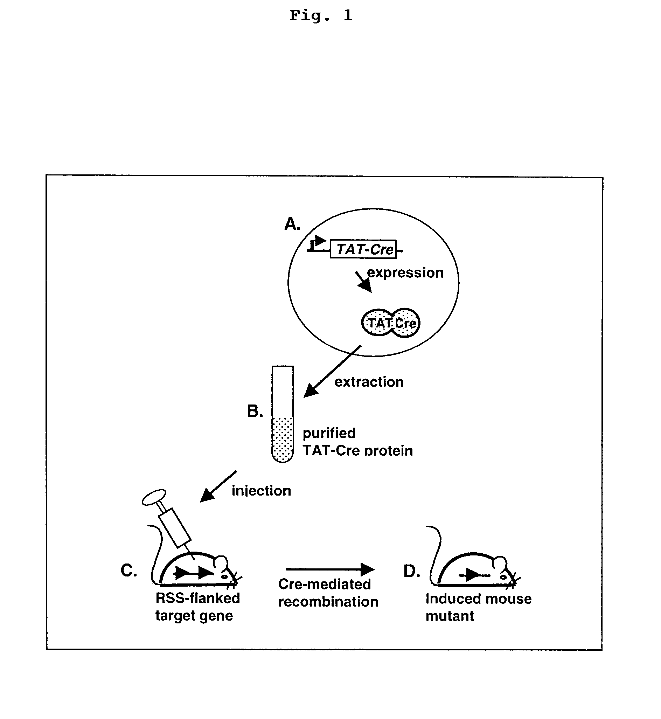

Figure 1: Generation of induced mouse mutants using purified fusion proteins.
A: Expression of the fusion protein consisting of the site-specific DNA recombinase (e.g. Cre) and the protein transduction domain (e.g. the HIV derived TAT peptide) in prokaryontic or eukaryontic cells.
B: Extraction and purification of the expressed fusion protein (e.g. as described in Nagahara et al., 1998).
C: Injection of the purified fusion protein into mice carrying the RRS-flanked target sequence.
D: Analysis of the pattern of induced target gene recombination and the resulting phenotype.
Triangle: RRS.

The expression "target sequences" according to the present invention means all kind of sequences which may be mutated (viz. deleted, translocated, integrated and/or inverted) by the action of the recombinase. The number of RRS in the target sequence depends on the kind of mutation to be performed by the recombinase. For most of the mutations (especially for deletions and invertions) two RRS are required which are flanking the sequence to be mutated (deleted or inverted). For some kinds of integrations only one RRS may be necessary within the target sequence.

The "living organisms" according to the present invention are multi-cell organisms and can be vertebrates such as mammals (e.g., rodents such as mice or rats) or non-mammals (e.g., fish) or can be invertebrates such as insects or worms. Most preferred living organisms are mice and fish.

The site-specific DNA recombinase domain within the fusion protein of the invention of the present application is preferably selected from a recombinase protein derived from Cre, Flp, φC31 recombinase (Thorpe and Smith, Proc. Natl. Acad. Sci, USA, vol. 95, 5505-5510 (1998)) and R recombinase (Araki et al., J. Mol. Biol., 182, 191-203 (1985)). The preferred recombinases are Cre (e.g., the Cre variant of aa 15 to 357 of SEQ ID NO: 2 or aa 325-667 of SEQ ID NO: 6) and Flpe (i.e., the Flp variant of aa 15 to 437 of SEQ ID NO: 4 or aa 325 to 747 of SEQ ID NO: 8).

The protein transduction domain according to the present invention is preferably derived from the Antennapedia protein of Drosophila, from the VP22 protein of HSV or from the TAT protein of HIV. Preferrably the protein transduction domain is derived from the TAT protein among which a TAT protein comprising the amino acid sequence shown in SEQ ID NO: 10 is most preferred.

The fusion of the two domains of the fusion protein can occur at any possible position, i.e., the protein transduction domain can be fused to the N- or C-terminal of the site-specific DNA recombinase or can be fused to active sites within the site-specific DNA recombinase. Preferrably the protein transfusion domain is fused to the N-terminal of the site-specific DNA recombinase domain.

The protein transduction domain can be fused to the site-specific DNA recombinase either through a direct chemical bond or through a linker molecule. Such linker molecule can be any bivalent chemical structure capable of linking the two domains. The preferred linker molecule according to the present invention is a short peptide, e.g., having 1 to 20, preferrably 1 to 10, amino acid residues. Specifically preferred short peptides are essentially consisting of Gly, Ala and/or Leu.

The fusion protein of the invention of the present application may further comprise other functional sequences such as secretion conferring signals, nuclear localization signals and/or signals conferring protein stabilization.

In case the fusion protein comprises a protein transduction domain derived from the TAT protein of HIV, the DNA sequence coding for said fusion protein preferrably comprises the sequence

5’ TAC GGC CGC AAG AAG CGC CGC CAA CGC CGC CGC 3’.

Such a preferred DNA sequence is for instance shown in SEQ ID NO: 11. In said sequence the 3' terminal codon ggc codes for the linker Gly. The DNA sequence of a suitable recombinase may be directly attached to said codon ggc.

The fusion protein can be obtained by the following steps:
1. Fusion of the recombinase coding region (e.g. encoding Cre: see amino acids 15 to 357 of SEQ ID NO: 2) with the sequence conferring protein translocation (e.g. the sequence encoding the TAT peptide YGRKKRRQRRR, SEQ ID NO: 10) using standard cloning protocols (Maniatis et al., Cold Spring Harbor Laboratory, New York (1989)) or chemical synthesis.
2. Generation of a construct for the expression of the fusion protein in prokaryotic or eukaryotic cells, e.g. in E. coli DH5a (Hanahan, J. Mol. Biol.;166(4):557-80 (1983)) using the QlAexpress pQE vector (Qiagen, Hilden).
3. Expression of the above mentioned fusion protein in prokaryotic or eukaryotic cells, e.g. in E. coli DH5a (Hanahan, 1983)
4. Extraction and purification of the above mentioned fusion protein e.g. as described in Nagahara et al., Nat. Med., 4(12):1449-52 (1998).

Injection of the purified fusion protein into a living organism (e.g., a mouse) carrying a gene comprising the RRS-flanked target sequence (e.g., in an amount of 5 to 50 µg per g body weight). To demonstrate the feasibility of the invention, a reporter mouse strain is used harbouring a RRS-flanked flanked cassette which, when deleted by the recombinase, allows the expression of a cellular marker protein, such as β-galacosidase (Thorey et al., Mol. Cell Biol., 18(10):6164 (1998)).

Analysis is achieved by determining the pattern of induced target gene recombination (e.g. through Southern blot analysis and X-Gal staining on tissue sections; Maniatis et al., 1989; Gossler and Zachgo, Joyner AL (Ed.), Oxford Univerity Press, Oxford, New York (1993)).

The procedure's advantages over current technology are as follows:
(i) The absence of background recombination before administration of the fusion protein.
(ii) The reduction of time and resources which are necessary to combine the recombinase transgene and two copies of the RRS-flanked target gene by conventional breeding.

## Claims

1. Use of a fusion protein comprising
(a) a site-specific DNA recombinase domain and
(b) a protein transduction domain
for preparing an agent for inducing target gene alterations in a living organism, wherein said living organism carries at least one or more recognition sites for said site-specific DNA recombinase integrated in its genome.

2. The use of claim 1 wherein the site-specific DNA recombinase domain is selected from a recombinase protein derived from Cre, Flp, φC31 recombinase, and R recombinase and preferably is Cre having amino acids 15 to 357 of SEQ ID NO: 2 or Flpe having amino acids 15 to 437 of SEQ ID NO: 4.

3. The use of claim 1 or 2 wherein the protein transduction domain is a protein derived from the Antennapedia protein of Drosophila, from the VP22 protein of HSV or from the TAT protein of HIV, and preferably is derived from the TAT protein.

4. The use of claim 3, wherein the TAT protein comprises the amino acid sequence

YGRKKRQRRR (SEQ ID NO: 10).

5. The use of claims 1 to 4, wherein the protein transduction domain is fused to the N-terminal of the site-specific DNA recombinase domain.

6. The use of claims 1 to 5, wherein the protein transduction domain is fused to the site-specific DNA recombinase domain through a direct chemical bond or through a linker molecule.

7. The use of claim 6, wherein the linker molecule is a short peptide having 1 to 20, preferably 1 to 10 amino acid residues.

8. The use of claims 1 to 7, wherein said fusion protein further comprises additional functional sequences.

9. The use of claim 1, wherein the fusion protein has the sequence shown in SEQ ID NOs: 2, 4, 6 or 8.

10. The use of claims 1 to 8, wherein the living organism is a vertebrate, preferably a rodent or a fish.

11. A method for inducing gene alterations in a living organism which comprises administering to said living organism, a fusion protein comprising a site-specific DNA recombinase domain and a protein transduction domain as defined in claims 1 to 9, wherein said living organism carries at least one or more recognition sites for said site-specific DNA recombinase integrated in its genome.

12. A fusion protein comprising
(a) a site-specific DNA recombinase domain and
(b) a protein transduction domain
provided that when (a) is Flp then (b) is not the VP22 protein of HSV.

13. The fusion protein of claim 11 being as defined in claims 2 to 9.

14. The fusion of claim 12 or 13, wherein the protein transduction domain is derived from the TAT protein of HIV.

15. A DNA sequence coding for the fusion protein of claim 12.

16. The DNA sequence of claim 15 comprising the sequence shown in SEQ ID NOs: 9 and/or 11.

17. A vector comprising the DNA sequence of claim 15.

18. A host cell transformed with the vector of claim 17 and/or comprising the DNA of claim 15.

19. A method for producing the fusion protein of claim 11 which comprises culturing the transformed host cell of claim 17 and isolating the fusion protein.

20. An injectable composition comprising the fusion protein as defined in claims 1 to 9 or12 to14.


SEQUENCE LISTING

<110> ARTEMIS Pharmaceuticals GmbH

<120> Transduction of recombinases for inducible
      gene targeting

<130> 992928ep/JH/ml

<140>
<141>

<160> 12

<170> PatentIn Ver. 2.1


<210> 1
<211> 1074
<212> DNA
<213> Artificial Sequence

<220>
<223> Description of Artificial Sequence: DNA sequence
      coding for a fusion protein TAT-Cre

<220>
<221> CDS
<222> (1)..(1071)

<400> 1
atg ggc tac ggc cgc aag aag cgc cgc caa cgc cgc cgc ggc atg tcc     48
Met Gly Tyr Gly Arg Lys Lys Arg Arg Gln Arg Arg Arg Gly Met Ser
  1               5                  10                  15

aat tta ctg acc gta cac caa aat ttg cct gca tta ccg gtc gat gca     96
Asn Leu Leu Thr Val His Gln Asn Leu Pro Ala Leu Pro Val Asp Ala
             20                  25                  30

acg agt gat gag gtt cgc aag aac ctg atg gac atg ttc agg gat cgc     144
Thr Ser Asp Glu Val Arg Lys Asn Leu Met Asp Met Phe Arg Asp Arg
         35                  40                  45

cag gcg ttt tct gag cat acc tgg aaa atg ctt ctg tcc gtt tgc cgg     192
Gln Ala Phe Ser Glu His Thr Trp Lys Met Leu Leu Ser Val Cys Arg
     50                  55                  60

tcg tgg gcg gca tgg tgc aag ttg aat aac cgg aaa tgg ttt ccc gca     240
Ser Trp Ala Ala Trp Cys Lys Leu Asn Asn Arg Lys Trp Phe Pro Ala
 65                  70                  75                  80

gaa cct gaa gat gtt cgc gat tat ctt cta tat ctt cag gcg cgc ggt     288
Glu Pro Glu Asp Val Arg Asp Tyr Leu Leu Tyr Leu Gln Ala Arg Gly
                 85                  90                  95

ctg gca gta aaa act atc cag caa cat ttg ggc cag cta aac atg ctt     336
Leu Ala Val Lys Thr Ile Gln Gln His Leu Gly Gln Leu Asn Met Leu
            100                 105                 110

cat cgt cgg tcc ggg ctg cca cga cca agt gac agc aat gct gtt tca 384
His Arg Arg Ser Gly Leu Pro Arg Pro Ser Asp Ser Asn Ala Val Ser
        115                 120                 125

ctg gtt atg cgg cgg atc cga aaa gaa aac gtt gat gcc ggt gaa cgt 432
Leu Val Met Arg Arg Ile Arg Lys Glu Asn Val Asp Ala Gly Glu Arg
    130                 135                 140

gca aaa cag gct cta gcg ttc gaa cgc act gat ttc gac cag gtt cgt 480
Ala Lys Gln Ala Leu Ala Phe Glu Arg Thr Asp Phe Asp Gln Val Arg
145                 150                 155                 160

tca ctc atg gaa aat agc gat cgc tgc cag gat ata cgt aat ctg gca 528
Ser Leu Met Glu Asn Ser Asp Arg Cys Gln Asp Ile Arg Asn Leu Ala
                165                 170                 175

ttt ctg ggg att gct tat aac acc ctg tta cgt ata gcc gaa att gcc 576
Phe Leu Gly Ile Ala Tyr Asn Thr Leu Leu Arg Ile Ala Glu Ile Ala
            180                 185                 190

agg atc agg gtt aaa gat atc tca cgt act gac ggt ggg aga atg tta 624
Arg Ile Arg Val Lys Asp Ile Ser Arg Thr Asp Gly Gly Arg Met Leu
        195                 200                 205

atc cat att ggc aga acg aaa acg ctg gtt agc acc gca ggt gta gag 672
Ile His Ile Gly Arg Thr Lys Thr Leu Val Ser Thr Ala Gly Val Glu
    210                 215                 220

aag gca ctt agc ctg ggg gta act aaa ctg gtc gag cga tgg att tcc 720
Lys Ala Leu Ser Leu Gly Val Thr Lys Leu Val Glu Arg Trp Ile Ser
225                 230                 235                 240

gtc tct ggt gta gct gat gat ccg aat aac tac ctg ttt tgc cgg gtc 768
Val Ser Gly Val Ala Asp Asp Pro Asn Asn Tyr Leu Phe Cys Arg Val
                245                 250                 255

aga aaa aat ggt gtt gcc gcg cca tct gcc acc agc cag cta tca act 816
Arg Lys Asn Gly Val Ala Ala Pro Ser Ala Thr Ser Gln Leu Ser Thr
            260                 265                 270

cgc gcc ctg gaa ggg att ttt gaa gca act cat cga ttg att tac ggc 864
Arg Ala Leu Glu Gly Ile Phe Glu Ala Thr His Arg Leu Ile Tyr Gly
        275                 280                 285

gct aag gat gac tct ggt cag aga tac ctg gcc tgg tct gga cac agt 912
Ala Lys Asp Asp Ser Gly Gln Arg Tyr Leu Ala Trp Ser Gly His Ser
    290                 295                 300

gcc cgt gtc gga gcc gcg cga gat atg gcc cgc gct gga gtt tca ata 960
Ala Arg Val Gly Ala Ala Arg Asp Met Ala Arg Ala Gly Val Ser Ile
305                 310                 315                 320

ccg gag atc atg caa gct ggt ggc tgg acc aat gta aat att gtc atg 1008
Pro Glu Ile Met Gln Ala Gly Gly Trp Thr Asn Val Asn Ile Val Met
                325                 330                 335

aac tat atc cgt aac ctg gat agt gaa aca ggg gca atg gtg cgc ctg 1056
Asn Tyr Ile Arg Asn Leu Asp Ser Glu Thr Gly Ala Met Val Arg Leu
            340                 345                 350

ctg gaa gat ggc gat tag                                                1074
Leu Glu Asp Gly Asp
        355

<210> 2
<211> 357
<212> PRT
<213> Artificial Sequence
<223> Description of Artificial Sequence: DNA sequence
      coding for a fusion protein TAT-Cre

<400> 2
Met Gly Tyr Gly Arg Lys Lys Arg Arg Gln Arg Arg Arg Gly Met Ser
  1               5                  10                  15

Asn Leu Leu Thr Val His Gln Asn Leu Pro Ala Leu Pro Val Asp Ala
             20                  25                  30

Thr Ser Asp Glu Val Arg Lys Asn Leu Met Asp Met Phe Arg Asp Arg
         35                  40                  45

Gln Ala Phe Ser Glu His Thr Trp Lys Met Leu Leu Ser Val Cys Arg
     50                  55                  60

Ser Trp Ala Ala Trp Cys Lys Leu Asn Asn Arg Lys Trp Phe Pro Ala
 65                  70                  75                  80

Glu Pro Glu Asp Val Arg Asp Tyr Leu Leu Tyr Leu Gln Ala Arg Gly
                 85                  90                  95

Leu Ala Val Lys Thr Ile Gln Gln His Leu Gly Gln Leu Asn Met Leu
            100                 105                 110

His Arg Arg Ser Gly Leu Pro Arg Pro Ser Asp Ser Asn Ala Val Ser
        115                 120                 125

Leu Val Met Arg Arg Ile Arg Lys Glu Asn Val Asp Ala Gly Glu Arg
    130                 135                 140

Ala Lys Gln Ala Leu Ala Phe Glu Arg Thr Asp Phe Asp Gln Val Arg
145                 150                 155                 160

Ser Leu Met Glu Asn Ser Asp Arg Cys Gln Asp Ile Arg Asn Leu Ala
                165                 170                 175

Phe Leu Gly Ile Ala Tyr Asn Thr Leu Leu Arg Ile Ala Glu Ile Ala
            180                 185                 190

Arg Ile Arg Val Lys Asp Ile Ser Arg Thr Asp Gly Gly Arg Met Leu
        195                 200                 205

Ile His Ile Gly Arg Thr Lys Thr Leu Val Ser Thr Ala Gly Val Glu
    210                 215                 220

Lys Ala Leu Ser Leu Gly Val Thr Lys Leu Val Glu Arg Trp Ile Ser
225                 230                 235                 240

Val Ser Gly Val Ala Asp Asp Pro Asn Asn Tyr Leu Phe Cys Arg Val
                245                 250                 255

Arg Lys Asn Gly Val Ala Ala Pro Ser Ala Thr Ser Gln Leu Ser Thr
            260                 265                 270

Arg Ala Leu Glu Gly Ile Phe Glu Ala Thr His Arg Leu Ile Tyr Gly
        275                 280                 285

Ala Lys Asp Asp Ser Gly Gln Arg Tyr Leu Ala Trp Ser Gly His Ser
    290                 295                 300

Ala Arg Val Gly Ala Ala Arg Asp Met Ala Arg Ala Gly Val Ser Ile
305                 310                 315                 320

Pro Glu Ile Met Gln Ala Gly Gly Trp Thr Asn Val Asn Ile Val Met
                325                 330                 335

Asn Tyr Ile Arg Asn Leu Asp Ser Glu Thr Gly Ala Met Val Arg Leu
            340                 345                 350

Leu Glu Asp Gly Asp
        355


<210> 3
<211> 1317
<212> DNA
<213> Artificial Sequence

<220>
<223> Description of Artificial Sequence: DNA sequence
      coding for a fusion protein TAT-Flpe

<220>
<221> CDS
<222> (1)..(1311)

<400> 3
atg ggc tac ggc cgc aag aag cgc cgc caa cgc cgc cgc ggc atg agt      48
Met Gly Tyr Gly Arg Lys Lys Arg Arg Gln Arg Arg Arg Gly Met Ser
  1               5                  10                  15

caa ttt gat ata tta tgt aaa aca cca cct aag gtc ctg gtt cgt cag      96
Gln Phe Asp Ile Leu Cys Lys Thr Pro Pro Lys Val Leu Val Arg Gln
             20                  25                  30

ttt gtg gaa agg ttt gaa aga cct tca ggg gaa aaa ata gca tca tgt     144
Phe Val Glu Arg Phe Glu Arg Pro Ser Gly Glu Lys Ile Ala Ser Cys
         35                  40                  45

gct gct gaa cta acc tat tta tgt tgg atg att act cat aac gga aca     192
Ala Ala Glu Leu Thr Tyr Leu Cys Trp Met Ile Thr His Asn Gly Thr
     50                  55                  60

gca atc aag aga gcc aca ttc atg agc tat aat act atc ata agc aat     240
Ala Ile Lys Arg Ala Thr Phe Met Ser Tyr Asn Thr Ile Ile Ser Asn
 65                  70                  75                  80

tcg ctg agt ttc gat att gtc aac aaa tca ctc cag ttt aaa tac aag     288
Ser Leu Ser Phe Asp Ile Val Asn Lys Ser Leu Gln Phe Lys Tyr Lys
                 85                  90                  95

acg caa aaa gca aca att ctg gaa gcc tca tta aag aaa tta att cct 336
Thr Gln Lys Ala Thr Ile Leu Glu Ala Ser Leu Lys Lys Leu Ile Pro
            100                 105                 110

gct tgg gaa ttt aca att att cct tac aat gga caa aaa cat caa tct 384
Ala Trp Glu Phe Thr Ile Ile Pro Tyr Asn Gly Gln Lys His Gln Ser
        115                 120                 125

gat atc act gat att gta agt agt ttg caa tta cag ttc gaa tca tcg 432
Asp Ile Thr Asp Ile Val Ser Ser Leu Gln Leu Gln Phe Glu Ser Ser
    130                 135                 140

gaa gaa gca gat aag gga aat agc cac agt aaa aaa atg ctt aaa gca 480
Glu Glu Ala Asp Lys Gly Asn Ser His Ser Lys Lys Met Leu Lys Ala
145                 150                 155                 160

ctt cta agt gag ggt gaa agc atc tgg gag atc act gag aaa ata cta 528
Leu Leu Ser Glu Gly Glu Ser Ile Trp Glu Ile Thr Glu Lys Ile Leu
                165                 170                 175

aat tcg ttt gag tat acc tcg aga ttt aca aaa aca aaa act tta tac 576
Asn Ser Phe Glu Tyr Thr Ser Arg Phe Thr Lys Thr Lys Thr Leu Tyr
            180                 185                 190

caa ttc ctc ttc cta gct act ttc atc aat tgt gga aga ttc agc gat 624
Gln Phe Leu Phe Leu Ala Thr Phe Ile Asn Cys Gly Arg Phe Ser Asp
        195                 200                 205

att aag aac gtt gat ccg aaa tca ttt aaa tta gtc caa aat aag tat 672
Ile Lys Asn Val Asp Pro Lys Ser Phe Lys Leu Val Gln Asn Lys Tyr
    210                 215                 220

ctg gga gta ata atc cag tgt tta gtg aca gag aca aag aca agc gtt 720
Leu Gly Val Ile Ile Gln Cys Leu Val Thr Glu Thr Lys Thr Ser Val
225                 230                 235                 240

agt agg cac ata tac ttc ttt agc gca agg ggt agg atc gat cca ctt 768
Ser Arg His Ile Tyr Phe Phe Ser Ala Arg Gly Arg Ile Asp Pro Leu
                245                 250                 255

gta tat ttg gat gaa ttt ttg agg aat tct gaa cca gtc cta aaa cga 816
Val Tyr Leu Asp Glu Phe Leu Arg Asn Ser Glu Pro Val Leu Lys Arg
            260                 265                 270

gta aat agg acc ggc aat tct tca agc aac aaa cag gaa tac caa tta 864
Val Asn Arg Thr Gly Asn Ser Ser Ser Asn Lys Gln Glu Tyr Gln Leu
        275                 280                 285

tta aaa gat aac tta gtc aga tcg tac aac aag gct ttg aag aaa aat 912
Leu Lys Asp Asn Leu Val Arg Ser Tyr Asn Lys Ala Leu Lys Lys Asn
    290                 295                 300

gcg cct tat cca atc ttt gct ata aag aat ggc cca aaa tct cac att 960
Ala Pro Tyr Pro Ile Phe Ala Ile Lys Asn Gly Pro Lys Ser His Ile
305                 310                 315                 320

gga aga cat ttg atg acc tca ttt ctg tca atg aag ggc cta acg gag 1008
Gly Arg His Leu Met Thr Ser Phe Leu Ser Met Lys Gly Leu Thr Glu
                325                 330                 335

ttg act aat gtt gtg gga aat tgg agc gat aag cgt gct tct gcc gtg 1056
Leu Thr Asn Val Val Gly Asn Trp Ser Asp Lys Arg Ala Ser Ala Val
            340                 345                 350

gcc agg aca acg tat act cat cag ata aca gca ata cct gat cac tac 1104
Ala Arg Thr Thr Tyr Thr His Gln Ile Thr Ala Ile Pro Asp His Tyr
        355                 360                 365

ttc gca cta gtt tct cgg tac tat gca tat gat cca ata tca aag gaa 1152
Phe Ala Leu Val Ser Arg Tyr Tyr Ala Tyr Asp Pro Ile Ser Lys Glu
    370                 375                 380

atg ata gca ttg aag gat gag act aat cca att gag gag tgg cag cat 1200
Met Ile Ala Leu Lys Asp Glu Thr Asn Pro Ile Glu Glu Trp Gln His
385                 390                 395                 400

ata gaa cag cta aag ggt agt gct gaa gga agc ata cga tac ccc gca 1248
Ile Glu Gln Leu Lys Gly Ser Ala Glu Gly Ser Ile Arg Tyr Pro Ala
                405                 410                 415

tgg aat ggg ata ata tca cag gag gta cta gac tac ctt tca tcc tac 1296
Trp Asn Gly Ile Ile Ser Gln Glu Val Leu Asp Tyr Leu Ser Ser Tyr
            420                 425                 430

ata aat aga cgc ata taatga 1317
Ile Asn Arg Arg Ile
        435

<210> 4
<211> 437
<212> PRT
<213> Artificial Sequence
<223> Description of Artificial Sequence: DNA sequence
coding for a fusion protein TAT-Flpe

<400> 4
Met Gly Tyr Gly Arg Lys Lys Arg Arg Gln Arg Arg Arg Gly Met Ser
1               5                   10                  15

Gln Phe Asp Ile Leu Cys Lys Thr Pro Pro Lys Val Leu Val Arg Gln
            20                  25                  30

Phe Val Glu Arg Phe Glu Arg Pro Ser Gly Glu Lys Ile Ala Ser Cys
        35                  40                  45

Ala Ala Glu Leu Thr Tyr Leu Cys Trp Met Ile Thr His Asn Gly Thr
    50                  55                  60

Ala Ile Lys Arg Ala Thr Phe Met Ser Tyr Asn Thr Ile Ile Ser Asn
65                  70                  75                  80

Ser Leu Ser Phe Asp Ile Val Asn Lys Ser Leu Gln Phe Lys Tyr Lys
                85                  90                  95

Thr Gln Lys Ala Thr Ile Leu Glu Ala Ser Leu Lys Lys Leu Ile Pro
            100                 105                 110

Ala Trp Glu Phe Thr Ile Ile Pro Tyr Asn Gly Gln Lys His Gln Ser
        115                 120                 125

Asp Ile Thr Asp Ile Val Ser Ser Leu Gln Leu Gln Phe Glu Ser Ser
    130                 135                 140

Glu Glu Ala Asp Lys Gly Asn Ser His Ser Lys Lys Met Leu Lys Ala
145                 150                 155                 160

Leu Leu Ser Glu Gly Glu Ser Ile Trp Glu Ile Thr Glu Lys Ile Leu
                165                 170                 175

Asn Ser Phe Glu Tyr Thr Ser Arg Phe Thr Lys Thr Lys Thr Leu Tyr
            180                 185                 190

Gln Phe Leu Phe Leu Ala Thr Phe Ile Asn Cys Gly Arg Phe Ser Asp
        195                 200                 205

Ile Lys Asn Val Asp Pro Lys Ser Phe Lys Leu Val Gln Asn Lys Tyr
    210                 215                 220

Leu Gly Val Ile Ile Gln Cys Leu Val Thr Glu Thr Lys Thr Ser Val
225                 230                 235                 240

Ser Arg His Ile Tyr Phe Phe Ser Ala Arg Gly Arg Ile Asp Pro Leu
                245                 250                 255

Val Tyr Leu Asp Glu Phe Leu Arg Asn Ser Glu Pro Val Leu Lys Arg
            260                 265                 270

Val Asn Arg Thr Gly Asn Ser Ser Ser Asn Lys Gln Glu Tyr Gln Leu
        275                 280                 285

Leu Lys Asp Asn Leu Val Arg Ser Tyr Asn Lys Ala Leu Lys Lys Asn
    290                 295                 300

Ala Pro Tyr Pro Ile Phe Ala Ile Lys Asn Gly Pro Lys Ser His Ile
305                 310                 315                 320

Gly Arg His Leu Met Thr Ser Phe Leu Ser Met Lys Gly Leu Thr Glu
                325                 330                 335

Leu Thr Asn Val Val Gly Asn Trp Ser Asp Lys Arg Ala Ser Ala Val
            340                 345                 350

Ala Arg Thr Thr Tyr Thr His Gln Ile Thr Ala Ile Pro Asp His Tyr
        355                 360                 365

Phe Ala Leu Val Ser Arg Tyr Tyr Ala Tyr Asp Pro Ile Ser Lys Glu
    370                 375                 380

Met Ile Ala Leu Lys Asp Glu Thr Asn Pro Ile Glu Glu Trp Gln His
385                 390                 395                 400

Ile Glu Gln Leu Lys Gly Ser Ala Glu Gly Ser Ile Arg Tyr Pro Ala
                405                 410                 415

Trp Asn Gly Ile Ile Ser Gln Glu Val Leu Asp Tyr Leu Ser Ser Tyr
            420                 425                 430

Ile Asn Arg Arg Ile
        435

<210> 5
<211> 2004
<212> DNA
<213> Artificial Sequence

<220>
<223> Description of Artificial Sequence: DNA sequence
      coding for a fusion protein VP22-Cre

<220>
<221> CDS
<222> (1)..(2001)

<400> 5
atg acc tct cgc cgc tcc gtg aag tcg ggt ccg cgg gag gtt ccg cgc    48
Met Thr Ser Arg Arg Ser Val Lys Ser Gly Pro Arg Glu Val Pro Arg
  1               5                  10                  15

gat gag tac gag gat ctg tac tac acc ccg tct tca ggt atg gcg agt    96
Asp Glu Tyr Glu Asp Leu Tyr Tyr Thr Pro Ser Ser Gly Met Ala Ser
             20                  25                  30

ccc gat agt ccg cct gac acc tcc cgc cgt ggc gcc cta cag aca cgc    144
Pro Asp Ser Pro Pro Asp Thr Ser Arg Arg Gly Ala Leu Gln Thr Arg
         35                  40                  45

tcg cgc cag agg ggc gag gtc cgt ttc gtc cag tac gac gag tcg gat    192
Ser Arg Gln Arg Gly Glu Val Arg Phe Val Gln Tyr Asp Glu Ser Asp
     50                  55                  60

tat gcc ctc tac ggg ggc tcg tct tcc gaa gac gac gaa cac ccg gag    240
Tyr Ala Leu Tyr Gly Gly Ser Ser Ser Glu Asp Asp Glu His Pro Glu
 65                  70                  75                  80

gtc ccc cgg acg cgg cgt ccc gtt tcc ggg gcg gtt ttg tcc ggc ccg    288
Val Pro Arg Thr Arg Arg Pro Val Ser Gly Ala Val Leu Ser Gly Pro
                 85                  90                  95

ggg cct gcg cgg gcg cct ccg cca ccc gct ggg tcc gga ggg gcc gga    336
Gly Pro Ala Arg Ala Pro Pro Pro Pro Ala Gly Ser Gly Gly Ala Gly
            100                 105                 110

cgc aca ccc acc acc gcc ccc cgg gcc ccc cga acc cag cgg gtg gcg    384
Arg Thr Pro Thr Thr Ala Pro Arg Ala Pro Arg Thr Gln Arg Val Ala
        115                 120                 125

act aag gcc ccc gcg gcc ccg gcg gcg gag acc acc cgc ggc agg aaa    432
Thr Lys Ala Pro Ala Ala Pro Ala Ala Glu Thr Thr Arg Gly Arg Lys
    130                 135                 140

tcg gcc cag cca gaa tcc gcc gca ctc cca gac gcc ccc gcg tcg acg    480
Ser Ala Gln Pro Glu Ser Ala Ala Leu Pro Asp Ala Pro Ala Ser Thr
145                 150                 155                 160

gcg cca acc cga tcc aag aca ccc gcg cag ggg ctg gcc aga aag ctg     528
Ala Pro Thr Arg Ser Lys Thr Pro Ala Gln Gly Leu Ala Arg Lys Leu
                165                 170                 175

cac ttt agc acc gcc ccc cca aac ccc gac gcg cca tgg acc ccc cgg     576
His Phe Ser Thr Ala Pro Pro Asn Pro Asp Ala Pro Trp Thr Pro Arg
            180                 185                 190

gtg gcc ggc ttt aac aag cgc gtc ttc tgc gcc gcg gtc ggg cgc ctg     624
Val Ala Gly Phe Asn Lys Arg Val Phe Cys Ala Ala Val Gly Arg Leu
        195                 200                 205

gcg gcc atg cat gcc cgg atg gcg gcg gtc cag ctc tgg gac atg tcg     672
Ala Ala Met His Ala Arg Met Ala Ala Val Gln Leu Trp Asp Met Ser
    210                 215                 220

cgt ccg cgc aca gac gaa gac ctc aac gaa ctc ctt ggc atc acc acc     720
Arg Pro Arg Thr Asp Glu Asp Leu Asn Glu Leu Leu Gly Ile Thr Thr
225                 230                 235                 240

atc cgc gtg acg gtc tgc gag ggc aaa aac ctg ctt cag cgc gcc aac     768
Ile Arg Val Thr Val Cys Glu Gly Lys Asn Leu Leu Gln Arg Ala Asn
                245                 250                 255

gag ttg gtg aat cca gac gtg gtg cag gac gtc gac gcg gcc acg gcg     816
Glu Leu Val Asn Pro Asp Val Val Gln Asp Val Asp Ala Ala Thr Ala
            260                 265                 270

act cga ggg cgt tct gcg gcg tcg cgc ccc acc gag cga cct cga gcc     864
Thr Arg Gly Arg Ser Ala Ala Ser Arg Pro Thr Glu Arg Pro Arg Ala
        275                 280                 285

cca gcc cgc tcc gct tct cgc ccc aga cgg ccc gtc gag ggt acc gag     912
Pro Ala Arg Ser Ala Ser Arg Pro Arg Arg Pro Val Glu Gly Thr Glu
    290                 295                 300

ctc gga tcc act agt cca gtg tgg tgg aat tct gca gat atc cag cac     960
Leu Gly Ser Thr Ser Pro Val Trp Trp Asn Ser Ala Asp Ile Gln His
305                 310                 315                 320

agt ggc ggc cgc atg tcc aat tta ctg acc gta cac caa aat ttg cct     1008
Ser Gly Gly Arg Met Ser Asn Leu Leu Thr Val His Gln Asn Leu Pro
                325                 330                 335

gca tta ccg gtc gat gca acg agt gat gag gtt cgc aag aac ctg atg     1056
Ala Leu Pro Val Asp Ala Thr Ser Asp Glu Val Arg Lys Asn Leu Met
            340                 345                 350

gac atg ttc agg gat cgc cag gcg ttt tct gag cat acc tgg aaa atg     1104
Asp Met Phe Arg Asp Arg Gln Ala Phe Ser Glu His Thr Trp Lys Met
        355                 360                 365

ctt ctg tcc gtt tgc cgg tcg tgg gcg gca tgg tgc aag ttg aat aac     1152
Leu Leu Ser Val Cys Arg Ser Trp Ala Ala Trp Cys Lys Leu Asn Asn
    370                 375                 380

cgg aaa tgg ttt ccc gca gaa cct gaa gat gtt cgc gat tat ctt cta     1200
Arg Lys Trp Phe Pro Ala Glu Pro Glu Asp Val Arg Asp Tyr Leu Leu
385                 390                 395                 400

tat ctt cag gcg cgc ggt ctg gca gta aaa act atc cag caa cat ttg 1248
Tyr Leu Gln Ala Arg Gly Leu Ala Val Lys Thr Ile Gln Gln His Leu
                405                 410                 415

ggc cag cta aac atg ctt cat cgt cgg tcc ggg ctg cca cga cca agt 1296
Gly Gln Leu Asn Met Leu His Arg Arg Ser Gly Leu Pro Arg Pro Ser
            420                 425                 430

gac agc aat gct gtt tca ctg gtt atg cgg cgg atc cga aaa gaa aac 1344
Asp Ser Asn Ala Val Ser Leu Val Met Arg Arg Ile Arg Lys Glu Asn
        435                 440                 445

gtt gat gcc ggt gaa cgt gca aaa cag gct cta gcg ttc gaa cgc act 1392
Val Asp Ala Gly Glu Arg Ala Lys Gln Ala Leu Ala Phe Glu Arg Thr
    450                 455                 460

gat ttc gac cag gtt cgt tca ctc atg gaa aat agc gat cgc tgc cag 1440
Asp Phe Asp Gln Val Arg Ser Leu Met Glu Asn Ser Asp Arg Cys Gln
465                 470                 475                 480

gat ata cgt aat ctg gca ttt ctg ggg att gct tat aac acc ctg tta 1488
Asp Ile Arg Asn Leu Ala Phe Leu Gly Ile Ala Tyr Asn Thr Leu Leu
                485                 490                 495

cgt ata gcc gaa att gcc agg atc agg gtt aaa gat atc tca cgt act 1536
Arg Ile Ala Glu Ile Ala Arg Ile Arg Val Lys Asp Ile Ser Arg Thr
            500                 505                 510

gac ggt ggg aga atg tta atc cat att ggc aga acg aaa acg ctg gtt 1584
Asp Gly Gly Arg Met Leu Ile His Ile Gly Arg Thr Lys Thr Leu Val
        515                 520                 525

agc acc gca ggt gta gag aag gca ctt agc ctg ggg gta act aaa ctg 1632
Ser Thr Ala Gly Val Glu Lys Ala Leu Ser Leu Gly Val Thr Lys Leu
    530                 535                 540

gtc gag cga tgg att tcc gtc tct ggt gta gct gat gat ccg aat aac 1680
Val Glu Arg Trp Ile Ser Val Ser Gly Val Ala Asp Asp Pro Asn Asn
545                 550                 555                 560

tac ctg ttt tgc cgg gtc aga aaa aat ggt gtt gcc gcg cca tct gcc 1728
Tyr Leu Phe Cys Arg Val Arg Lys Asn Gly Val Ala Ala Pro Ser Ala
                565                 570                 575

acc agc cag cta tca act cgc gcc ctg gaa ggg att ttt gaa gca act 1776
Thr Ser Gln Leu Ser Thr Arg Ala Leu Glu Gly Ile Phe Glu Ala Thr
            580                 585                 590

cat cga ttg att tac ggc gct aag gat gac tct ggt cag aga tac ctg 1824
His Arg Leu Ile Tyr Gly Ala Lys Asp Asp Ser Gly Gln Arg Tyr Leu
        595                 600                 605

gcc tgg tct gga cac agt gcc cgt gtc gga gcc gcg cga gat atg gcc 1872
Ala Trp Ser Gly His Ser Ala Arg Val Gly Ala Ala Arg Asp Met Ala
    610                 615                 620

cgc gct gga gtt tca ata ccg gag atc atg caa gct ggt ggc tgg acc 1920

Arg Ala Gly Val Ser Ile Pro Glu Ile Met Gln Ala Gly Gly Trp Thr
625                 630                 635                 640

aat gta aat att gtc atg aac tat atc cgt aac ctg gat agt gaa aca     1968
Asn Val Asn Ile Val Met Asn Tyr Ile Arg Asn Leu Asp Ser Glu Thr
                645                 650                 655

ggg gca atg gtg cgc ctg ctg gaa gat ggc gat tag                     2004
Gly Ala Met Val Arg Leu Leu Glu Asp Gly Asp
            660                 665

<210> 6
<211> 667
<212> PRT
<213> Artificial Sequence
<223> Description of Artificial Sequence: DNA sequence
      coding for a fusion protein VP22-Cre

<400> 6
Met Thr Ser Arg Arg Ser Val Lys Ser Gly Pro Arg Glu Val Pro Arg
  1               5                  10                  15

Asp Glu Tyr Glu Asp Leu Tyr Tyr Thr Pro Ser Ser Gly Met Ala Ser
             20                  25                  30

Pro Asp Ser Pro Pro Asp Thr Ser Arg Arg Gly Ala Leu Gln Thr Arg
         35                  40                  45

Ser Arg Gln Arg Gly Glu Val Arg Phe Val Gln Tyr Asp Glu Ser Asp
     50                  55                  60

Tyr Ala Leu Tyr Gly Gly Ser Ser Ser Glu Asp Asp Glu His Pro Glu
 65                  70                  75                  80

Val Pro Arg Thr Arg Arg Pro Val Ser Gly Ala Val Leu Ser Gly Pro
                 85                  90                  95

Gly Pro Ala Arg Ala Pro Pro Pro Pro Ala Gly Ser Gly Gly Ala Gly
            100                 105                 110

Arg Thr Pro Thr Thr Ala Pro Arg Ala Pro Arg Thr Gln Arg Val Ala
        115                 120                 125

Thr Lys Ala Pro Ala Ala Pro Ala Ala Glu Thr Thr Arg Gly Arg Lys
    130                 135                 140

Ser Ala Gln Pro Glu Ser Ala Ala Leu Pro Asp Ala Pro Ala Ser Thr
145                 150                 155                 160

Ala Pro Thr Arg Ser Lys Thr Pro Ala Gln Gly Leu Ala Arg Lys Leu
                165                 170                 175

His Phe Ser Thr Ala Pro Pro Asn Pro Asp Ala Pro Trp Thr Pro Arg
            180                 185                 190

Val Ala Gly Phe Asn Lys Arg Val Phe Cys Ala Ala Val Gly Arg Leu
        195                 200                 205

Ala Ala Met His Ala Arg Met Ala Ala Val Gln Leu Trp Asp Met Ser
    210                 215                 220

Arg Pro Arg Thr Asp Glu Asp Leu Asn Glu Leu Leu Gly Ile Thr Thr
225                 230                 235                 240

Ile Arg Val Thr Val Cys Glu Gly Lys Asn Leu Leu Gln Arg Ala Asn
                245                 250                 255

Glu Leu Val Asn Pro Asp Val Val Gln Asp Val Asp Ala Ala Thr Ala
            260                 265                 270

Thr Arg Gly Arg Ser Ala Ala Ser Arg Pro Thr Glu Arg Pro Arg Ala
        275                 280                 285

Pro Ala Arg Ser Ala Ser Arg Pro Arg Arg Pro Val Glu Gly Thr Glu
    290                 295                 300

Leu Gly Ser Thr Ser Pro Val Trp Trp Asn Ser Ala Asp Ile Gln His
305                 310                 315                 320

Ser Gly Gly Arg Met Ser Asn Leu Leu Thr Val His Gln Asn Leu Pro
                325                 330                 335

Ala Leu Pro Val Asp Ala Thr Ser Asp Glu Val Arg Lys Asn Leu Met
            340                 345                 350

Asp Met Phe Arg Asp Arg Gln Ala Phe Ser Glu His Thr Trp Lys Met
        355                 360                 365

Leu Leu Ser Val Cys Arg Ser Trp Ala Ala Trp Cys Lys Leu Asn Asn
    370                 375                 380

Arg Lys Trp Phe Pro Ala Glu Pro Glu Asp Val Arg Asp Tyr Leu Leu
385                 390                 395                 400

Tyr Leu Gln Ala Arg Gly Leu Ala Val Lys Thr Ile Gln Gln His Leu
                405                 410                 415

Gly Gln Leu Asn Met Leu His Arg Arg Ser Gly Leu Pro Arg Pro Ser
            420                 425                 430

Asp Ser Asn Ala Val Ser Leu Val Met Arg Arg Ile Arg Lys Glu Asn
        435                 440                 445

Val Asp Ala Gly Glu Arg Ala Lys Gln Ala Leu Ala Phe Glu Arg Thr
    450                 455                 460

Asp Phe Asp Gln Val Arg Ser Leu Met Glu Asn Ser Asp Arg Cys Gln
465                 470                 475                 480

Asp Ile Arg Asn Leu Ala Phe Leu Gly Ile Ala Tyr Asn Thr Leu Leu
                485                 490                 495

Arg Ile Ala Glu Ile Ala Arg Ile Arg Val Lys Asp Ile Ser Arg Thr
            500                 505                 510

Asp Gly Gly Arg Met Leu Ile His Ile Gly Arg Thr Lys Thr Leu Val

        515                 520                 525

Ser Thr Ala Gly Val Glu Lys Ala Leu Ser Leu Gly Val Thr Lys Leu
    530                 535                 540

Val Glu Arg Trp Ile Ser Val Ser Gly Val Ala Asp Asp Pro Asn Asn
545                 550                 555                 560

Tyr Leu Phe Cys Arg Val Arg Lys Asn Gly Val Ala Ala Pro Ser Ala
                565                 570                 575

Thr Ser Gln Leu Ser Thr Arg Ala Leu Glu Gly Ile Phe Glu Ala Thr
            580                 585                 590

His Arg Leu Ile Tyr Gly Ala Lys Asp Asp Ser Gly Gln Arg Tyr Leu
        595                 600                 605

Ala Trp Ser Gly His Ser Ala Arg Val Gly Ala Ala Arg Asp Met Ala
    610                 615                 620

Arg Ala Gly Val Ser Ile Pro Glu Ile Met Gln Ala Gly Gly Trp Thr
625                 630                 635                 640

Asn Val Asn Ile Val Met Asn Tyr Ile Arg Asn Leu Asp Ser Glu Thr
                645                 650                 655

Gly Ala Met Val Arg Leu Leu Glu Asp Gly Asp
            660                 665


<210> 7
<211> 2004
<212> DNA
<213> Artificial Sequence

<220>
<223> Description of Artificial Sequence: DNA sequence
      coding for a fusion protein VP22-Flpe

<220>
<221> CDS
<222> (1)..(1998)

<400> 7
atg acc tct cgc cgc tcc gtg aag tcg ggt ccg cgg gag gtt ccg cgc    48
Met Thr Ser Arg Arg Ser Val Lys Ser Gly Pro Arg Glu Val Pro Arg
  1               5                  10                  15

gat gag tac gag gat ctg tac tac acc ccg tct tca ggt atg gcg agt    96
Asp Glu Tyr Glu Asp Leu Tyr Tyr Thr Pro Ser Ser Gly Met Ala Ser
             20                  25                  30

ccc gat agt ccg cct gac acc tcc cgc cgt ggc gcc cta cag aca cgc    144
Pro Asp Ser Pro Pro Asp Thr Ser Arg Arg Gly Ala Leu Gln Thr Arg
         35                  40                  45

tcg cgc cag agg ggc gag gtc cgt ttc gtc cag tac gac gag tcg gat    192
Ser Arg Gln Arg Gly Glu Val Arg Phe Val Gln Tyr Asp Glu Ser Asp
     50                  55                  60

tat gcc ctc tac ggg ggc tcg tct tcc gaa gac gac gaa cac ccg gag 240
Tyr Ala Leu Tyr Gly Gly Ser Ser Ser Glu Asp Asp Glu His Pro Glu
65                  70                  75                  80

gtc ccc cgg acg cgg cgt ccc gtt tcc ggg gcg gtt ttg tcc ggc ccg 288
Val Pro Arg Thr Arg Arg Pro Val Ser Gly Ala Val Leu Ser Gly Pro
                85                  90                  95

ggg cct gcg cgg gcg cct ccg cca ccc gct ggg tcc gga ggg gcc gga 336
Gly Pro Ala Arg Ala Pro Pro Pro Pro Ala Gly Ser Gly Gly Ala Gly
            100                 105                 110

cgc aca ccc acc acc gcc ccc cgg gcc ccc cga acc cag cgg gtg gcg 384
Arg Thr Pro Thr Thr Ala Pro Arg Ala Pro Arg Thr Gln Arg Val Ala
        115                 120                 125

act aag gcc ccc gcg gcc ccg gcg gcg gag acc acc cgc ggc agg aaa 432
Thr Lys Ala Pro Ala Ala Pro Ala Ala Glu Thr Thr Arg Gly Arg Lys
    130                 135                 140

tcg gcc cag cca gaa tcc gcc gca ctc cca gac gcc ccc gcg tcg acg 480
Ser Ala Gln Pro Glu Ser Ala Ala Leu Pro Asp Ala Pro Ala Ser Thr
145                 150                 155                 160

gcg cca acc cga tcc aag aca ccc gcg cag ggg ctg gcc aga aag ctg 528
Ala Pro Thr Arg Ser Lys Thr Pro Ala Gln Gly Leu Ala Arg Lys Leu
                165                 170                 175

cac ttt agc acc gcc ccc cca aac ccc gac gcg cca tgg acc ccc cgg 576
His Phe Ser Thr Ala Pro Pro Asn Pro Asp Ala Pro Trp Thr Pro Arg
            180                 185                 190

gtg gcc ggc ttt aac aag cgc gtc ttc tgc gcc gcg gtc ggg cgc ctg 624
Val Ala Gly Phe Asn Lys Arg Val Phe Cys Ala Ala Val Gly Arg Leu
        195                 200                 205

gcg gcc atg cat gcc cgg atg gcg gcg gtc cag ctc tgg gac atg tcg 672
Ala Ala Met His Ala Arg Met Ala Ala Val Gln Leu Trp Asp Met Ser
    210                 215                 220

cgt ccg cgc aca gac gaa gac ctc aac gaa ctc ctt ggc atc acc acc 720
Arg Pro Arg Thr Asp Glu Asp Leu Asn Glu Leu Leu Gly Ile Thr Thr
225                 230                 235                 240

atc cgc gtg acg gtc tgc gag ggc aaa aac ctg ctt cag cgc gcc aac 768
Ile Arg Val Thr Val Cys Glu Gly Lys Asn Leu Leu Gln Arg Ala Asn
                245                 250                 255

gag ttg gtg aat cca gac gtg gtg cag gac gtc gac gcg gcc acg gcg 816
Glu Leu Val Asn Pro Asp Val Val Gln Asp Val Asp Ala Ala Thr Ala
            260                 265                 270

act cga ggg cgt tct gcg gcg tcg cgc ccc acc gag cga cct cga gcc 864
Thr Arg Gly Arg Ser Ala Ala Ser Arg Pro Thr Glu Arg Pro Arg Ala
        275                 280                 285

cca gcc cgc tcc gct tct cgc ccc aga cgg ccc gtc gag ggt acc gag 912
Pro Ala Arg Ser Ala Ser Arg Pro Arg Arg Pro Val Glu Gly Thr Glu
    290                 295                 300

ctc gga tcc act agt cca gtg tgg tgg aat tct gca gat atc cag cac 960
Leu Gly Ser Thr Ser Pro Val Trp Trp Asn Ser Ala Asp Ile Gln His
305                 310                 315                 320

agt ggc ggc cgc atg tcc aat tta ctg acc gta cac caa aat ttg cct 1008
Ser Gly Gly Arg Met Ser Asn Leu Leu Thr Val His Gln Asn Leu Pro
                325                 330                 335

gca tta ccg gtc gat gca acg agt gat gag gtt cgc aag aac ctg atg 1056
Ala Leu Pro Val Asp Ala Thr Ser Asp Glu Val Arg Lys Asn Leu Met
            340                 345                 350

gac atg ttc agg gat cgc cag gcg ttt tct gag cat acc tgg aaa atg 1104
Asp Met Phe Arg Asp Arg Gln Ala Phe Ser Glu His Thr Trp Lys Met
        355                 360                 365

ctt ctg tcc gtt tgc cgg tcg tgg gcg gca tgg tgc aag ttg aat aac 1152
Leu Leu Ser Val Cys Arg Ser Trp Ala Ala Trp Cys Lys Leu Asn Asn
    370                 375                 380

cgg aaa tgg ttt ccc gca gaa cct gaa gat gtt cgc gat tat ctt cta 1200
Arg Lys Trp Phe Pro Ala Glu Pro Glu Asp Val Arg Asp Tyr Leu Leu
385                 390                 395                 400

tat ctt cag gcg cgc ggt ctg gca gta aaa act atc cag caa cat ttg 1248
Tyr Leu Gln Ala Arg Gly Leu Ala Val Lys Thr Ile Gln Gln His Leu
                405                 410                 415

ggc cag cta aac atg ctt cat cgt cgg tcc ggg ctg cca cga cca agt 1296
Gly Gln Leu Asn Met Leu His Arg Arg Ser Gly Leu Pro Arg Pro Ser
            420                 425                 430

gac agc aat gct gtt tca ctg gtt atg cgg cgg atc cga aaa gaa aac 1344
Asp Ser Asn Ala Val Ser Leu Val Met Arg Arg Ile Arg Lys Glu Asn
        435                 440                 445

gtt gat gcc ggt gaa cgt gca aaa cag gct cta gcg ttc gaa cgc act 1392
Val Asp Ala Gly Glu Arg Ala Lys Gln Ala Leu Ala Phe Glu Arg Thr
    450                 455                 460

gat ttc gac cag gtt cgt tca ctc atg gaa aat agc gat cgc tgc cag 1440
Asp Phe Asp Gln Val Arg Ser Leu Met Glu Asn Ser Asp Arg Cys Gln
465                 470                 475                 480

gat ata cgt aat ctg gca ttt ctg ggg att gct tat aac acc ctg tta 1488
Asp Ile Arg Asn Leu Ala Phe Leu Gly Ile Ala Tyr Asn Thr Leu Leu
                485                 490                 495

cgt ata gcc gaa att gcc agg atc agg gtt aaa gat atc tca cgt act 1536
Arg Ile Ala Glu Ile Ala Arg Ile Arg Val Lys Asp Ile Ser Arg Thr
            500                 505                 510

gac ggt ggg aga atg tta atc cat att ggc aga acg aaa acg ctg gtt 1584
Asp Gly Gly Arg Met Leu Ile His Ile Gly Arg Thr Lys Thr Leu Val
        515                 520                 525

agc acc gca ggt gta gag aag gca ctt agc ctg ggg gta act aaa ctg 1632
Ser Thr Ala Gly Val Glu Lys Ala Leu Ser Leu Gly Val Thr Lys Leu
    530                 535                 540

gtc gag cga tgg att tcc gtc tct ggt gta gct gat gat ccg aat aac     1680
Val Glu Arg Trp Ile Ser Val Ser Gly Val Ala Asp Asp Pro Asn Asn
545                 550                 555                 560

tac ctg ttt tgc cgg gtc aga aaa aat ggt gtt gcc gcg cca tct gcc     1728
Tyr Leu Phe Cys Arg Val Arg Lys Asn Gly Val Ala Ala Pro Ser Ala
                565                 570                 575

acc agc cag cta tca act cgc gcc ctg gaa ggg att ttt gaa gca act     1776
Thr Ser Gln Leu Ser Thr Arg Ala Leu Glu Gly Ile Phe Glu Ala Thr
            580                 585                 590

cat cga ttg att tac ggc gct aag gat gac tct ggt cag aga tac ctg     1824
His Arg Leu Ile Tyr Gly Ala Lys Asp Asp Ser Gly Gln Arg Tyr Leu
        595                 600                 605

gcc tgg tct gga cac agt gcc cgt gtc gga gcc gcg cga gat atg gcc     1872
Ala Trp Ser Gly His Ser Ala Arg Val Gly Ala Ala Arg Asp Met Ala
    610                 615                 620

cgc gct gga gtt tca ata ccg gag atc atg caa gct ggt ggc tgg acc     1920
Arg Ala Gly Val Ser Ile Pro Glu Ile Met Gln Ala Gly Gly Trp Thr
625                 630                 635                 640

aat gta aat att gtc atg aac tat atc cgt aac ctg gat agt gaa aca     1968
Asn Val Asn Ile Val Met Asn Tyr Ile Arg Asn Leu Asp Ser Glu Thr
                645                 650                 655

ggg gca atg gtg cgc ctg ctg gaa gat ggc gattag                      2004
Gly Ala Met Val Arg Leu Leu Glu Asp Gly
            660                 665

<210> 8
<211> 666
<212> PRT
<213> Artificial Sequence
<223> Description of Artificial Sequence: DNA sequence
      coding for a fusion protein VP22-Flpe

<400> 8
Met Thr Ser Arg Arg Ser Val Lys Ser Gly Pro Arg Glu Val Pro Arg
  1               5                  10                  15

Asp Glu Tyr Glu Asp Leu Tyr Tyr Thr Pro Ser Ser Gly Met Ala Ser
             20                  25                  30

Pro Asp Ser Pro Pro Asp Thr Ser Arg Arg Gly Ala Leu Gln Thr Arg
         35                  40                  45

Ser Arg Gln Arg Gly Glu Val Arg Phe Val Gln Tyr Asp Glu Ser Asp
     50                  55                  60

Tyr Ala Leu Tyr Gly Gly Ser Ser Ser Glu Asp Asp Glu His Pro Glu
 65                  70                  75                  80

Val Pro Arg Thr Arg Arg Pro Val Ser Gly Ala Val Leu Ser Gly Pro

                85                  90                  95

Gly Pro Ala Arg Ala Pro Pro Pro Pro Ala Gly Ser Gly Gly Ala Gly
            100                 105                 110

Arg Thr Pro Thr Thr Ala Pro Arg Ala Pro Arg Thr Gln Arg Val Ala
        115                 120                 125

Thr Lys Ala Pro Ala Ala Pro Ala Ala Glu Thr Thr Arg Gly Arg Lys
    130                 135                 140

Ser Ala Gln Pro Glu Ser Ala Ala Leu Pro Asp Ala Pro Ala Ser Thr
145                 150                 155                 160

Ala Pro Thr Arg Ser Lys Thr Pro Ala Gln Gly Leu Ala Arg Lys Leu
                165                 170                 175

His Phe Ser Thr Ala Pro Pro Asn Pro Asp Ala Pro Trp Thr Pro Arg
            180                 185                 190

Val Ala Gly Phe Asn Lys Arg Val Phe Cys Ala Ala Val Gly Arg Leu
        195                 200                 205

Ala Ala Met His Ala Arg Met Ala Ala Val Gln Leu Trp Asp Met Ser
    210                 215                 220

Arg Pro Arg Thr Asp Glu Asp Leu Asn Glu Leu Leu Gly Ile Thr Thr
225                 230                 235                 240

Ile Arg Val Thr Val Cys Glu Gly Lys Asn Leu Leu Gln Arg Ala Asn
                245                 250                 255

Glu Leu Val Asn Pro Asp Val Val Gln Asp Val Asp Ala Ala Thr Ala
            260                 265                 270

Thr Arg Gly Arg Ser Ala Ala Ser Arg Pro Thr Glu Arg Pro Arg Ala
        275                 280                 285

Pro Ala Arg Ser Ala Ser Arg Pro Arg Arg Pro Val Glu Gly Thr Glu
    290                 295                 300

Leu Gly Ser Thr Ser Pro Val Trp Trp Asn Ser Ala Asp Ile Gln His
305                 310                 315                 320

Ser Gly Gly Arg Met Ser Asn Leu Leu Thr Val His Gln Asn Leu Pro
                325                 330                 335

Ala Leu Pro Val Asp Ala Thr Ser Asp Glu Val Arg Lys Asn Leu Met
            340                 345                 350

Asp Met Phe Arg Asp Arg Gln Ala Phe Ser Glu His Thr Trp Lys Met
        355                 360                 365

Leu Leu Ser Val Cys Arg Ser Trp Ala Ala Trp Cys Lys Leu Asn Asn
    370                 375                 380

Arg Lys Trp Phe Pro Ala Glu Pro Glu Asp Val Arg Asp Tyr Leu Leu
385                 390                 395                 400

Tyr Leu Gln Ala Arg Gly Leu Ala Val Lys Thr Ile Gln Gln His Leu
                405                 410                 415

Gly Gln Leu Asn Met Leu His Arg Arg Ser Gly Leu Pro Arg Pro Ser
            420                 425                 430

Asp Ser Asn Ala Val Ser Leu Val Met Arg Arg Ile Arg Lys Glu Asn
        435                 440                 445

Val Asp Ala Gly Glu Arg Ala Lys Gln Ala Leu Ala Phe Glu Arg Thr
    450                 455                 460

Asp Phe Asp Gln Val Arg Ser Leu Met Glu Asn Ser Asp Arg Cys Gln
465                 470                 475                 480

Asp Ile Arg Asn Leu Ala Phe Leu Gly Ile Ala Tyr Asn Thr Leu Leu
                485                 490                 495

Arg Ile Ala Glu Ile Ala Arg Ile Arg Val Lys Asp Ile Ser Arg Thr
            500                 505                 510

Asp Gly Gly Arg Met Leu Ile His Ile Gly Arg Thr Lys Thr Leu Val
        515                 520                 525

Ser Thr Ala Gly Val Glu Lys Ala Leu Ser Leu Gly Val Thr Lys Leu
    530                 535                 540

Val Glu Arg Trp Ile Ser Val Ser Gly Val Ala Asp Asp Pro Asn Asn
545                 550                 555                 560

Tyr Leu Phe Cys Arg Val Arg Lys Asn Gly Val Ala Ala Pro Ser Ala
                565                 570                 575

Thr Ser Gln Leu Ser Thr Arg Ala Leu Glu Gly Ile Phe Glu Ala Thr
            580                 585                 590

His Arg Leu Ile Tyr Gly Ala Lys Asp Asp Ser Gly Gln Arg Tyr Leu
        595                 600                 605

Ala Trp Ser Gly His Ser Ala Arg Val Gly Ala Ala Arg Asp Met Ala
    610                 615                 620

Arg Ala Gly Val Ser Ile Pro Glu Ile Met Gln Ala Gly Gly Trp Thr
625                 630                 635                 640

Asn Val Asn Ile Val Met Asn Tyr Ile Arg Asn Leu Asp Ser Glu Thr
                645                 650                 655

Gly Ala Met Val Arg Leu Leu Glu Asp Gly
            660                 665


<210> 9
<211> 33
<212> DNA
<213> Human immunodeficiency virus

<400> 9
tacggccgca agaagcgccg ccaacgccgc cgc                                33

<210> 10
<211> 11
<212> PRT
<213> Human immunodeficiency virus

<400> 10
Tyr Gly Arg Lys Lys Arg Arg Gln Arg Arg Arg
1               5                   10


<210> 11
<211> 42
<212> DNA
<213> Human immunodeficiency virus

<220>
<221> CDS
<222> (4)..(42)

<400> 11
atg ggc tac ggc cgc aag aag cgc cgc caa cgc cgc cgc ggc            42
    Gly Tyr Gly Arg Lys Lys Arg Arg Gln Arg Arg Arg Gly
    1               5                   10


<210> 12
<211> 13
<212> PRT
<213> Human immunodeficiency virus

<400> 12
Gly Tyr Gly Arg Lys Lys Arg Arg Gln Arg Arg Arg Gly
1               5                   10